**Europäisches Patentamt**

**European Patent Office**

**Office européen des brevets**

(11) Numéro de publication: **0 414 980 A1**

## DEMANDE DE BREVET EUROPEEN

(21) Numéro de dépôt: **89440090.2**

(51) Int. Cl.5: **A61K 7/16**

(22) Date de dépôt: **31.08.89**

Le titre de l'invention a été modifié (Directives relatives à l'examen pratiqué à l'OEB, A-III, 7.3)

(43) Date de publication de la demande:
**06.03.91 Bulletin 91/10**

(84) Etats contractants désignés:
**AT BE CH DE ES FR GB GR IT LI LU NL SE**

(71) Demandeur: **Dana, Dominique**
**Le Mas du Cade Quartier des Plaines**
**F-83440 Tanneron(FR)**

(72) Inventeur: **Dana, Dominique**
**Le Mas du Cade Quartier des Plaines**
**F-83440 Tanneron(FR)**

(74) Mandataire: **Bossard, Jacques-René**
**Cabinet MEYER & COURTASSOL Bureau**
**EUROPE 20 Place des Halles**
**F-67000 Strasbourg(FR)**

(54) **Composition de dentifrice.**

(57) Composition dentifrice liquide, caractérisée en ce qu'elle contient comme ingrédients essentiels:
- au moins un composant antiseptique et/ou antimicrobien
- au moins un composant agissant sur les impuretés, notamment le tartre, et
- au moins un composant constituant une source de fluor, ainsi que les adjuvants usuels, à savoir, de préférence un colorant, un parfum, un astringent et./ou un agent de brillance
- le tout étant en solution aqueuse, présentée dans un récipient équipé de moyens de projection d'un jet nébulisé, soit par un système mécanique à pompage et refoulement, soit par un gaz propulseur comprimé.

## NOUVELLE COMPOSITION DE DENTIFRICE.

La présente invention concerne une nouvelle composition de dentifrice ainsi qu'une nouvelle présentation et un nouveau mode d'utilisation d'une telle composition.

A ce jour, les différentes compositions de dentifrices se présentent sous forme soit d'une pâte ou d'un gel, soit d'une poudre, soit d'une solution, dont, dans chaque cas, on doit prélever une certaine quantité et l'appliquer sur les dents au moyen d'une brosse. De plus en plus couramment, on trouve des tubes de dentifrices munis de doseurs, contenant ou non des gaz propulseurs, mais dont l'application ne dispense pas de l'utilisation d'une brosse à dents.

L'usage d'une telle brosse représente toujours une contrainte pour de multiples raisons ; c'est entre autres pourquoi il est difficile d'obtenir des enfants qu'ils se brossent régulièrement les dents ; de même, hors de chez soi, il n'est pas possible de se nettoyer les dents, après un repas ou avant une visite ; en allant plus loin, c'est aussi pourquoi il n'est pas non plus possible de nettoyer les dents des animaux, notamment des chiens.

L'invention élimine ces inconvénients grâce à une composition dentifrice liquide présentée dans un récipient permettant de la projeter soit sous forme de brouillard ou de fines gouttelettes (spray), soit sous forme d'un jet sous pression. L'innovation essentielle de la présente invention consiste en l'utilisation sous forme de spray d'une solution liquide à usage dentifrice, qui est active sur la plaque dentaire et agit en prévention des caries et du tartre grâce à ses composants.

Il convient de noter que l'on a déjà désigné des compositions dentifrices sous la dénomination de "spray" ; toutefois cette dénomination était abusive et fallacieuse car en fait elle désignait seulement un système d'expulsion et de dosage approximatif d'une composition en pâte ou en gel, sans aucun effet de projection de cette composition ni de réduction simultanée en fines particules, ni de projection sans aucune forme, ce qui rendait encore indispensable l'emploi d'une brosse. Dans certains cas, les sprays proposés avaient cependant trait à des solutions que l'on pouvait pulvériser dans la cavité buccale que des produits destinés aux soins des dents ou à la prévention des caries.

Au contraire, la pulvérisation de la composition selon l'invention a pour effet d'en projeter une multitude de particules liquides sur toutes les dents, par simple orientation appropriée du jet de gouttelettes, de déposer sur toutes les dents certains composants de la solution de l'invention, qui ont des propriétés bactéricides ou qui protègent contre les caries.

Cette projection peut se faire au moyen d'un dispositif du genre vaporisateur classique, c'est-à-dire comportant un système du type à pompage et à refoulement vers l'extérieur par un ajutage de pulvérisation.

Elle peut également se faire au moyen d'un gaz propulseur sous pression, de préférence de l'azote.

Dans les deux cas, selon une réalisation préférentielle de l'invention, le jet de dentifrice liquide est expulsé sous une pression relativement élevée, de manière à associer aux effets résultant de la nature des constituants de la composition des effets mécaniques de nettoyage des dents et de leurs interstices.

On peut d'ailleurs associer à un récipient contenant la composition de dentifrice liquide un autre récipient contenant de l'eau de rinçage, laquelle est également expulsée sous forme d'un jet sous pression. De tels récipients peuvent être conçus sous forme de recharges interchangeables s'adaptant au système d'expulsion sous pression.

Ce système peut lui-même comporter un embout basculable assurant le caractère direction du jet.

Le principe de base de l'invention réside dans l'association de trois constituants principaux:

- un complexe enzymatique (dont certains composants sont issus de la salive) possédant des propriétés bactéricides naturelles et une activité anti-plaque directe,
- des fluors aminés dont l'effet est immédiat par fixation du fluor sur l'émail des dents dès les premières secondes. De plus les amines restent fixées en surface de la dent, contribuant à la prévention contre les caries et constituant une pellicule protectrice qui s'oppose au dépôt de la plaque dentaire,
- de la Diméthicone possédant un effet filmogène sur les dents et retardant l'apparition du tartre et le dépôt de la nicotine, rendant ainsi aux dents un aspect lisse et brillant.

On rajoute à ces constituants majeurs un certain nombre de composants tels que:

- du savon tensio-actif à effet nettoyant
- des sucres non carriogènes (par exemple du type Sorbitol, Xylitol)
- du menthol parfumant la solution
- de l'eau, un conservateur, de l'alcool...

D'une manière plus générale, une composition selon l'invention comprend:

- au moins un composant antiseptique et/ou antimicrobien,

-au moins un composant agissant sur les impuretés, notamment le tartre,
- au moins un composant constituant une source de fluor,
- ainsi que de préférence un colorant, un parfum, un astringent et./ou un agent de brillance,
- le tout étant en solution aqueuse.

A titre d'exemple, on donne ci-après une composition de dentifrice selon l'invention, répondant aux principes posés auparavant, et apte à être appliquée sur les dents sous forme d'un jet nébulisé.

On donne, en pourcentage pondéral, des intervalles dans lesquels se situent les valeurs à hauteur desquelles les composants entrent dans la solution. En particulier pour les trois composants principaux, il y a plusieurs possibilités de constituants et on se borne à en évoquer un ou deux exemples précis :

## Exemple :

|  |  |
| --- | --- |
| - Complexe enzymatique | [1,5-10] |
| - Amines fluorées | [0,5 - 5] |
| (Fluorure de cetylamine) | |

| (DihydroFluorure de Bis (Hydroxyethyl) Aminopropyl N Hydroxyethyl octadecylamine) | |
| --- | --- |
| - Dimethicone | [0,1 - 3] |
| - Polysorbate 80 | [0 - 3] |
| - Polysorbate 20 | [0 - 3] |
| - Saccharine | [0 - 2] |
| - Glycérine | [0 - 30] |
| - Sorbitol | [0 - 60] |
| - Xylitol | [0 - 10] |
| - Thiocyanate | [0 - 1] |
| - Silice colloïdale | [0 - 3] |
| - Alcool | [0 - 5] |
| - $H_2O$ | [0 - 60] |
| - Parfums (menthe-anis) | [0,5 - 3] |
| - Savons | [0 - 10] |
| - Conservateur | [0 -2] |
| - Antiseptique | [0 -2] |

Selon un exemple préférentiel, la composition est constituée des ingrédients suivants:

| - Complexe enzymatique | [4,6] |
|---|---|
| - Amines fluorées | [1,9] |
| - Dimethicone | [1,5] |
| - Polysorbate 80 | [1,4] |
| - Polysorbate 20 | [1,4] |
| - Saccharine | [0,15] |
| - Glycérine | [10] |
| - Sorbitol | [40] |
| - Xylitol | [5] |
| - Thiocyanate | [0,1] |
| - Silice colloïdale | [1] |
| - Eau | QSP [30,3] |
| - Parfums (menthe-anis) | [1] |
| - Savons | [1,5] |
| - Conservateur | [0,15] |

Cette composition, présentée en petit appareil de poche, peut être utilisée même dans les lieux publics à n'importe quel moment, y compris par des enfants, sans nécessiter de brosse. Elle peut être aussi présentée sous forme d'accessoire plus volumineux, destiné à être placé à demeure dans les salles de bain.

On peut également l'utiliser pour nettoyer les dents des animaux, tels les animaux de compagnie, par exemple les chiens, qui ne se prêtent évidemment pas à l'utilisation d'une brosse.

Les exemples de composition donnés ci-dessus ne sont évidemment pas limitatifs, mais illustrent la simplicité de telles compositions.

**Revendications**

1. Composition dentifrice liquide, caractérisée en ce qu'elle contient comme ingrédients essentiels:
- au moins un composant antiseptique et/ou antimicrobien
- au moins un composant agissant sur les impuretés, notamment le tartre, et
- au moins un composant constituant une source de fluor, ainsi que les adjuvants usuels, à savoir, de préférence un colorant, un parfum, un astringent et./ou un agent de brillance
- le tout étant en solution aqueuse, présentée dans un récipient équipé de moyens de projection d'un jet nébulisé, soit par un système mécanique à pompage et refoulement, soit par un gaz propulseur comprimé.

2. Composition selon la revendication 1, caractérisée en ce que le composant anti-microbien est l'hexetidine, le composant anti-tartre est le carbonate de calcium et le composant fluoré est le monofluorophosphate de sodium.

3. Composition selon la revendication 2, caractérisée en ce qu'elle contient les composants suivants, dont les pourcentages pondéraux sont pris dans les intervalles correspondants:

| - Complexe enzymatique | [1,5-10] |
|---|---|
| - Amines fluorées | [0,5 - 5] |
| - Dimethicone | [0,1 - 3] |
| - Polysorbate 80 | [0 - 3] |
| - Polysorbate 20 | [0 - 3] |
| - Saccharine | [0 - 2] |
| - Glycérine | [0 - 30] |
| - Sorbitol | [0 - 60] |
| - Xylitol | [0 - 10] |
| - Thiocyanate | [0 - 1] |
| - Silice colloïdale | [0 - 3] |
| - Alcool | [0 - 5] |
| - $H_2O$ | [0 - 60] |
| - Parfums (menthe-anis) | [0,5 - 3] |
| - Savons | [0 - 10] |
| - Conservateur | [0 - 2] |
| - Antiseptique | [0 - 2] |

4. Composition selon la revendication 2, caractérisée en ce qu'elle contient les composants suivants:

| - Complexe enzymatique | [4,6] |
|---|---|
| - Amines fluorées | [1,9] |
| - Dimethicone | [1,5] |
| - Polysorbate 80 | [1,4] |
| - Polysorbate 20 | [1,4] |
| - Saccharine | [0,15] |
| - Glycérine | [10] |
| - Sorbitol | [40] |
| - Xylitol | [5] |
| - Thiocyanate | [0,1] |
| - Silice colloïdale | [1] |
| - Eau | QSP [30,3] |
| - Parfums (menthe-anis) | [1] |
| - Savons | [1,5] |
| - Conservateur | [0,15] |

5. Composition selon les revendications 2 et 3, caractérisée en ce que les amines fluorées sont des fluorures de cetylamine.

6. Composition selon les revendications 2 et 3, caractérisée en ce que les amines fluorées sont composées de Dihydrofluorure de Bis (Hydroxyethyl) Aminopropyl N Hydroxyethyl octadécylamine.

7. Dispositif pour le nettoyage des dents, caractérisé en ce qu'il consiste en un récipient contenant une composition selon l'une des revendications 1 à 3, et équipé de moyens assurant l'expulsion de ladite composition sous forme d'un jet sous pression.

8. Dispositif selon la revendication 7, caractérisé en ce qu'il comporte également un récipient rempli d'eau de rinçage, pouvant être branché sur les moyens d'expulstion sous pression.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| E | WO-A-8 911 848 (DANA)<br>* En entier *<br>--- | 1,3-8 | A 61 K 7/16 |
| X | EP-A-0 049 830 (MUHLEMANN)<br>* Exemple III; revendications 1,2,6 *<br>--- | 1,7 | |
| A | EP-A-0 180 483 (RUTLAND CONSULTING GROUP LTD)<br>* En entier *<br>--- | 1-8 | |
| A | EP-A-0 208 009 (RICHARDSON GmbH)<br>* En entier *<br>--- | 1-8 | |
| E | FR-A-2 631 823 (DANA)<br>* En entier *<br>------ | 1-2,7-8 | |

DOMAINES TECHNIQUES RECHERCHES (Int. Cl.5)

A 61 K

Le présent rapport a été établi pour toutes les revendications

| Lieu de la recherche | Date d'achèvement de la recherche | Examinateur |
|---|---|---|
| LA HAYE | 08-05-1990 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant